## Europäisches Patentamt

## European Patent Office

## Office européen des brevets

(1) Publication number: **0 205 668**
**B1**

# EUROPEAN PATENT SPECIFICATION

(45) Date of publication of patent specification: **01.02.89**

(51) Int. Cl.⁴: **A 61 F 2/12,** A 61 F 2/02

(21) Application number: **85200947.1**

(22) Date of filing: **17.06.85**

(54) **Improved implantable prosthesis.**

(43) Date of publication of application:
**30.12.86 Bulletin 86/52**

(45) Publication of the grant of the patent:
**01.02.89 Bulletin 89/05**

(84) Designated Contracting States:
**DE FR GB NL**

(56) References cited:
**DE-B-2 457 041**
**FR-A-2 419 065**
**US-A-3 663 968**
**US-A-4 138 382**

(73) Proprietor: **Netto, Daniel J.**
**153 N. Vendome Street**
**Los Angeles California 90026 (US)**

(72) Inventor: **Netto, Daniel J.**
**153 N. Vendome Street**
**Los Angeles California 90026 (US)**

(74) Representative: **van der Beek, George Frans**
**et al**
**Nederlandsch Octrooibureau Johan de Wittlaan**
**15 P.O. Box 29720**
**NL-2502 LS The Hague (NL)**

Courier Press, Leamington Spa, England.

## Description

The invention relates to a prosthetic implant, and more particularly to the field of surgical prosthetic implants for human breasts or the like.

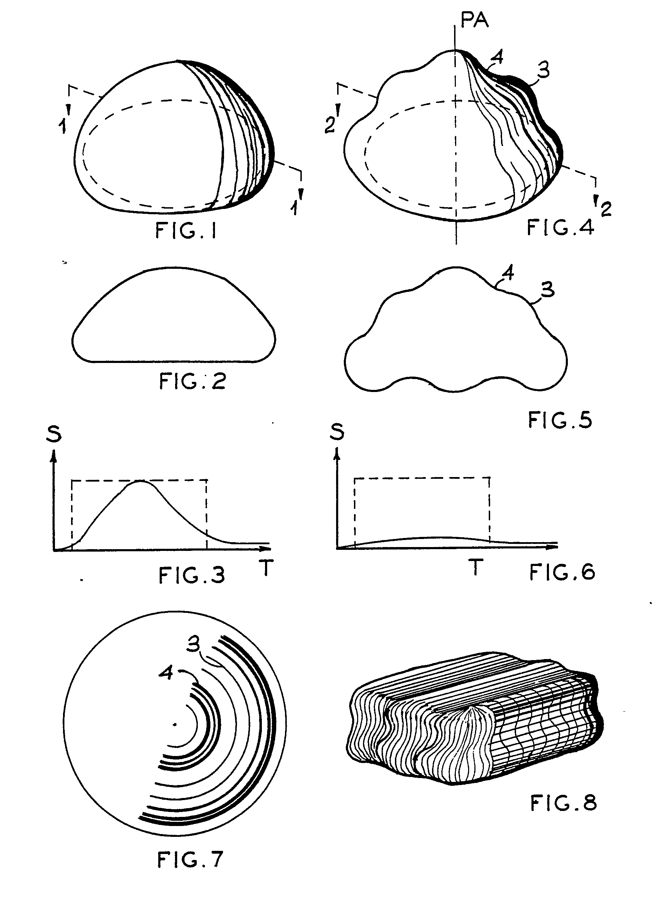

Such an implant is known from FR-A-2,419,065. This device consists of a continuous flexible shell filled with a kneadable material such as an elastomer or silicone. According to fig. 2 of this reference said device may have undulations over a substantial portion of its inwardly directed surface. This device can easily adapt to deformations of the body tissue lying under the implanted device. Another advantage of such a device is that it can be implanted easily into the body in order to correct body curves.

The volume of the kneadable material is a little smaller than the maximum volume of the device known from said reference.

A prosthetic human breast implant is surgically placed within the body to serve as breast replacement after mastectomy or to otherwise cosmetically improve the size and firmness thereof.

It is known to persons skilled in the art of prosthetic devices, especially plastic surgeons, that a connective tissue capsule formation grows around the implant after surgery. The contractile component of this capsule, the MYOFIBROBLAST, frequently causes capsular contracture which can lead to deformation of the implant and discomfort or acute pain in the patient.

Surgeons occasionally implant prosthetic devices in other parts of the body for cosmetic or reconstruction purposes, and a similar growth of connective tissue and resulting pressure and pain can occur when such a procedure is carried out.

If the prosthetic implant were designed to contract under pressure, then the pain would be alleviated. However, the solution to the problem is not feasible because the whole idea of implanting the prosthesis is to achieve a pre-determined increase in size or firmness in the breast or other part of the body, and a prosthesis that would shrink under pressure would fail to perform its intended function.

Some prosthetic devices are hollow, consisting of a silicone outer shell filled with fluid. It is known to persons skilled in the art that pain from the pressure exerted by the capsular contracture can be treated by injection of a growth-inhibiting drug into the fluid, which drug diffuses through the fluid and the silicone shell and then inhibits the capsular contracture. However, this treatment is only temporary and in many cases does not give adequate relief.

A careful study has been made of the problem of how to give permanent relief from discomfort and pain caused by the pressure of the connective tissue capsule contracture against the prosthetic implant. According to the present invention this objective is accomplished.

The present invention relates to a prosthetic implant comprising an outer shell, said implant being characterised in that the external surface thereof has a sinusoidal contour, the form of which is stable but when subjected to capsular contracture pressures the amplitude of the sinusoidal contour reduces, resulting in deformation of the shell without affecting the volume and firmness of the implant.

Preferably, said outer shell is a spherical segment and lines on the external surface joining points of equal height on the sinusoidal contour are concentric to the polar axis.

Furthermore, the implant according to the invention may comprise a sinusoidal contour along its spherical surface and bottom.

In particular, the implant according to the invention may embody a cubical or rectangular box configuration, in which lines on the external surface joining points of equal height on the sinusoidal contour run parallel to the edges on all sides.

In the present description the sinusoidal forms will be indicated as "waves".

The capsular contracture around the implant exerts pressure on the peaks of the waves but not on the troughs. As the pressure increases, the surface of the prosthesis deforms under this pressure; the peaks of the waves compress under the pressure, become shallower. In this fashion, the stress is relieved by action of the prosthetic device in changing its shape as the pressure increases. The patient is not uncomfortable, and yet the prosthetic device does not shrink or change its firmness.

It is an object of this invention to provide such a prosthesis which will maintain the desired size and firmness during the post operative period and after the reactivity of the myofibroblast has ceased.

The foregoing and other objects and advantages of this invention, will become apparent to those skilled in the art of prosthetic devices upon reviewing the drawings and reading the description of the preferred embodiment.

Figure 1 is a perspective view of a conventional human breast prosthetic implant. Figure 2 is a cross-section view of the device shown in figure 1. Figure 4 is a perspective view of an implant embodying the invention, a prosthetic implant with a surface comprising a series of sinusoidal waves along its latitude and bottom, concentric to the polar axis. Figure 5 is a cross-section view of the device shown in figure 4. Figure 7 is a top view of the device shown in figure 4, showing the concentric waves. Figure 8 is a perspective view of a cubical or rectangular box type prosthesis showing the waves running parallel to the edges. Figure 3 is a graph illustrating the stress occurring during the weeks following surgery, exerted on a conventional implant as illustrated in figure 1. Figure 6 is a graph, illustrating the stress occurring during the weeks following surgery, exerted on the invention, a prosthetic implant with a surface comprising a series of sinusoidal waves.

Figure 1 is a perspective view of a conventional human breast prosthetic implant, showing the smooth surface. A cross-section of the implant of figure 1, along the line 1-1, is shown in figure 2. As the pressure is exerted by capsular contracture across and around this device, the pressure builds up and can cause pain, as there is no way to relieve tlhe stress short of contraction of the implant. Figure 4 is a perspective view of a prosthetic implant embodying the invention. Figure 5 is a cross-section view along the line 2-2 of figure 4, and figure 7 is a top view. In the invention, as the capsule contracts around the implant, pressure is exerted on peaks 3 of the sinusoidal waves, but not on troughs 4. As the pressure increases with time, peaks 3 contract and troughs 4 expand, and as this process takes place, the amplitude of the waves gets smaller and the surface becomes less wavy. As a consequence, the path length of the wave is shortened and the capsule made to relax. The pressure eventually stops increasing, and the shape of the prosthetic implant then stops changing. At that time, the surface will be nearly smooth, and the remaining waves will be too small in amplitude to be noticeable. Another major improvement is the significant reduction of surface contact between the prosthesis and the surrounding tissue due to the wavy surface, improving the blood circulation. In figure 3, the stress caused by the contracture of the connective tissue capsule around a conventional implant is shown against time. During the weeks following the surgical implant, there is a large increase in stress, which can cause pain and discomfort. This critical period is shown within the dotted area. By contrast, figure 6 shows how the invention reduces the stress to such an extent that there will be no discomfort at all.

In the preferred embodiment, the invention is made of an outer shell of silicone and the interior is filled with a viscous fluid or gelatin-like material. The construction of the invention is sufficiently rigid that the implant will maintain its firmness except under the considerable pressure exerted by the capsular contracture, and under this pressure the peaks of the waves in the surface of the prosthesis slowly decrease in amplitude, and the troughs slowly expand outward due to the incompressibility of the fluid.

In an alternative embodiment, the implant being a cubical or rectangular box encasement, the waves run parallel to the edges as shown in figure 8.

The exact amplitude and length of the waves are not critical and will vary, depending on the required size and firmness of the implant as selected by the surgeon. However, on a human breast implant of average size, about 10 cm in diameter and 4 cm high, as an example, the wavelength will be about 1 cm to 2.5 cm, and the amplitude will be about 0.2 cm to 0.7 cm.

A method to manufacture said prosthesis is as follows: a mold is made in two parts, separable along the plane, at about half the height, perpendicular to the polar axis (PA). Then a mixture of silicone is poured into the mold and by centrifugal means, the mixture is kept against the outer periphery of the cavity. Once the mixture has cured, the mold is separated and the shell removed. The shell thickness is varied by the amount of mixture that is poured into the mold. By means of a syringe and needle, a fluid is injected into the prosthesis and the firmness obtained by the amount of fluid injected. The needle is then withdrawn and the puncture hole sealed with a silicone cement. For the solid mass prosthesis, the mold cavity is completely filled with the silicone mixture and the prosthesis removed after the mixture has cured. Although the invention has been described with reference to the example, it is to be understood that the invetion is not limited to the specific embodiment.

## Claims

1. A prosthetic implant comprising an outer shell, characterized in that the external surface thereof has a sinusoidal contour the form of which is stable but when subjected to capsular contracture pressures the amplitude of the sinusoidal contour reduces, resulting in deformation of the shell without affecting the volume and firmness of the implant.

2. A prosthetic implant according to claim 1, in which the shell is a spherical segment and lines on the external surface joining points of equal height on the sinusoidal contour are concentric to the polar axis.

3. A prosthetic implant according to claims 1 and 2, comprising a sinusoidal contour along its spherical surface and bottom. (fig. 5).

4. A prosthetic implant according to claim 1, embodying a cubical or rectangular box configuration, in which lines on the external surface joining points of equal height on the sinusoidal contour run parallel to the edges on all sides. (fig. 8).

## Patentansprüche

1. Prothetisches Implantat mit Aussenhülle, dadurch gekennzeichnet, dass ihre Aussenfläche eine sinusförmige Kontur hat, deren Form stabil ist, aber die Amplitude der sinusförmigen Kontur sich bei Einwirkung kapsulärer Kontrakturdrucke verringert, was eine Deformation der Aussenhülle zur Folge hat, ohne das Volumen und die Festigkeit des Implantats zu beeinträchtigen.

2. Prothetisches Implantat gemäss Anspruch 1, bei welchem die Aussenhülle ein kugelförmiges Segment ist und Linien auf der Aussenfläche, die Punkte gleicher Höhe auf der sinusförmigen Kontur verbinden, konzentrisch zur Polarachse verlaufen.

3. Prothetisches Implantat gemäss den Ansprüchen 1 und 2, das entlang seiner kugelförmigen Aussenfläche und seines Bodenteils eine sinusförmige Kontur aufweist (Fig. 5).

4. Prothetisches Implantat gemäss Anspruch 1, eine kubische oder rechteckige Kastenform darstellend, bei der Linien auf der Aussenfläche, die Punkte gleicher Höhe auf der sinusförmigen Kontur verbinden, parallel zu den Kanten auf allen Seiten verlaufen (Fig. 6).

## Revendications

1. Implant de prothèse comportant une enveloppe extérieure, caractérisé en ce que sa surface extérieure présente un contour sinusoïdal de forme stable mais dont l'amplitude diminue lorsqu'il est soumis à des pressions de contracture capsulaire, ce qui provoque une déformation de l'enveloppe qui n'affecte ni le volume ni la fermeté de l'implant.

2. Implant de prothèse conforme à la revendication 1, dans lequel l'enveloppe est une calotte sphérique et les lignes de la surface extérieure reliant les points d'égale hauteur du contour sinusoïdal sont centréessur l'axe polaire.

3. Implant de prothèse conforme aux revendications 1 et 2, comportant un contour sinusoïdal sur sa surface sphérique et sur son fond (figure 5).

4. Implant de prothèse conforme à la revendication 1, présentant une configuration de boîte cubique ou rectangulaire, dans lequel les lignes de la surface extérieure reliant les points d'égale hauteur sur le contour sinusoïdal sont parallèles aux bords des côtés (figure 8).

FIG. 1

FIG. 2

FIG. 3

FIG. 4

FIG. 5

FIG. 6

FIG. 7

FIG. 8